(19) Europäisches Patentamt
European Patent Office
Office européen des brevets

(11) **EP 4 134 029 A1**

(12) **EUROPEAN PATENT APPLICATION**

(43) Date of publication:
**15.02.2023 Bulletin 2023/07**

(21) Application number: **22184602.5**

(22) Date of filing: **13.07.2022**

(51) International Patent Classification (IPC):
**A61B 18/12** (2000.01)   **A61B 18/00** (2000.01)
**A61B 5/00** (1968.09)

(52) Cooperative Patent Classification (CPC):
**A61B 18/12; A61B 5/6843; A61B 5/6847;**
**A61B 5/7267; G16H 20/40; G16H 40/63;**
A61B 5/4836; A61B 2018/00577;
A61B 2018/00589; A61B 2018/00601;
A61B 2018/0069; A61B 2018/00702;
A61B 2018/0072; A61B 2018/00732;
A61B 2018/00761;                         (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **13.08.2021   CN 202110932338**

(71) Applicant: **Carbon (Shenzhen) Medical Device Co.,**
**Ltd.**
**Shenzhen City, Guangdong (CN)**

(72) Inventors:
• **ZHANG, Shiping**
  **Shenzhen City (CN)**
• **YANG, Xiaowei**
  **Shenzhen City (CN)**
• **FENG, Mingyu**
  **Shenzhen City (CN)**
• **WU, Menglin**
  **Shenzhen City (CN)**

(74) Representative: **Proi World Intellectual Property**
**GmbH**
**Obermattweg 12**
**6052 Hergiswil, Kanton Nidwalden (CH)**

(54) **METHOD AND SYSTEM FOR ARTIFICIAL INTELLIGENCE-BASED RADIOFREQUENCY ABLATION PARAMETER OPTIMIZATION AND INFORMATION SYNTHESIS**

(57)    A method and system for artificial intelligence-based radiofrequency ablation parameter optimization and information synthesis are provided. The method is applied to a radiofrequency ablation controller including a processor and an artificial intelligence module. The processor of the radiofrequency ablation controller preprocesses sample data and sends the preprocessed sample data to the artificial intelligence module. The artificial intelligence module establishes an artificial neural network model according to the preprocessed sample data and a radiofrequency ablation control parameter for the sample data. The processor preprocesses signals collected by sensors on a plasma wand. The artificial intelligence module imports preprocessed sensor data into the artificial neural network model for analysis and fusion, to obtain the radiofrequency ablation control parameter. According to environmental parameters, the present invention can provide information such as the position of a lesion tissue and the size of an ablation range with reference to an artificial neural network, which is helpful for doctors to determine the condition of the lesion tissue and control energy transfer during the ablation process, thereby helping to reduce secondary trauma.

FIG. 2

EP 4 134 029 A1

(52) Cooperative Patent Classification (CPC): (Cont.)
A61B 2018/00767; A61B 2018/00791;
A61B 2018/00875; A61B 2018/00904;
A61B 2560/0242; G06N 3/043

**Description**

**TECHNICAL FIELD**

The present invention relates to the field of radiofrequency ablation technologies, and specifically, to a method and system for artificial intelligence-based radiofrequency ablation parameter optimization and information synthesis.

**BACKGROUND**

Currently, existing radiofrequency ablation devices mainly focus on the generation of voltage and power, so that high-frequency electrical energy is successfully transferred to a target tissue of a patient. Moreover, the operation mainly relies on clinical experience of an attending doctor, and corresponding control parameters still lack an accurate thermal basis. The ablation process involves many disciplines such as biology, heat transfer, mechanics, and clinical medicine. Therefore, an ablation range needs to be controlled precisely, to reduce unnecessary damage as much as possible, and ensure that destruction of lesion cells is maximized without damaging normal tissue cells.

During surgery, threshold values of current and temperature are generally used to estimate and control energy transfer, to protect the patient and ensure the safe usage of medical instruments. However, measuring dynamic current and temperature cannot achieve real-time control, and is low in efficiency. Moreover, current ablation devices cannot achieve automatic closed-loop ablation or blood coagulationpower and energy configuration.

In addition, minimally invasive operation is high barriers medical techniqueand need a comparative long learning curve, meanwhile young doctors usually do not have sufficient knowledge and experience, furtherly training and guidance for those entry level operators at the beginning of surgical operation are particularly difficult. Existing training methods usually rely on knowledge explanations, video introductions, and the like, and long-term operating experience of doctors cannot be accurately expressed, which cannot well help beginners understand the operating steps and identify the operating environment. For the control of radiofrequency parameters, it needs to consider that decision outputs are aimed at different tissue types such as soft tissues, fat, and bones during treatment. The simple analysis of model and environment is insufficient to deal with complex real situations. Therefore, there is an urgent need for a method and system for information synthesis in clinical practice, so that an auxiliary environmental analysis can be performed in clinical practice and simulation training, and matching operating parameters can be provided to doctors for reference.

**SUMMARY**

An objective of the present invention is to provide a method and system for artificial intelligence-based radiofrequency ablation parameter optimization and information synthesis, to cope with the problem that current ablation devices cannot perform automatic configuration of powerand energy.

The technical scheme of the present invention is: the invention provide a method for artificial intelligence-based radiofrequency ablation parameter optimization and information synthesis, the method being applied to a radiofrequency ablation controller comprising a processor and an artificial intelligence module; and the method comprising

S1. preprocessing, by the processor of the radiofrequency ablation controller, sample data, wherein the sample data is sensor parameters of a target substance and a surrounding environment of the target substance, and sending, by the processor, the preprocessed sample data to the artificial intelligence module;

S2. performing, by the artificial intelligence module, fuzzy computing on the preprocessed sample data and radiofrequency ablation control parameters for the sample data, and establishing an artificial neural network model, wherein the control parameters comprise ablation occurrence time, ablation trigger time, and energy frequency;

S3. preprocessing, by the processor, signals collected by sensors on a plasma wand and sends the preprocessed signals to the artificial intelligence module; and

S4. importing, by the artificial intelligence module, preprocessed sensor data into the artificial neural network model established in the step S2 for analysis and fusion, to obtain the radiofrequency ablation control parameters.

Furthermore, the method being applied to the radiofrequency ablation controller, wherein a signal input end of the radiofrequency ablation controller is connected to a signal output end of each sensor on the plasma wand, and the radiofrequency ablation controller outputs a control parameter of the plasma wand.

Furthermore,the sensors comprise voltage, current, impedance, temperature, humidity, contact force, and the like.

Furthermore,in the steps S1 and S3, the preprocessing further comprises:

S1-1. amplifying and filtering sensor signal data;and

S1-2. performing signal adjustment comprising linear and nonlinear correction on the filtered signal data.

Furthermore,in the step S2, the fuzzy computing further comprises:

S2-1. constructing a sensor signal data set $S$ for the sample data; constructing a decision set of the controller; and constructing a decision matrix V according to an effect of elements in the signal data set Son an output of the decision set D;

S2-2. normalizing V, summing up each row, combining the summed data into a sensor coefficient matrix W, and normalizing W;

S2-3. for a comprehensive environment in which the sample data is located, constructing a corresponding relationship matrix R for a single environment respectively according to Cauchy distribution, wherein the quantity of the relationship matrices is k;

S2-4. fusing the sensor coefficient matrix W and the relationship matrices R of k single environments respectively, that is, F=WR, to obtain fusion results of k single collection environments, $[F_1, F_2, ..., F_k]$; and

S2-5. inputting the fusion results $[F_1, F_2, ..., F_k]$ of k single tissue environments of a plurality of groups of samples and the radiofrequency ablation control parameters corresponding to each sample into an artificial neural network according to the steps S2-1 to S2-4, and setting a quantity of layers of the network for self-learning, to obtain a single tissue environment factor corresponding to the sample data and the established artificial neural network model.

Furthermore,in the step S2, the fuzzy computing further comprises:
S2-1. constructing the sensor signal data set $S=[s_1, s_2, ..., s_n]$, wherein n is the quantity of sensors;
constructing the decision set $D=[d_1, d_2, ..., d_m]$ of the controller based on types andpropertyof the sensors, wherein m is the quantity of decision results, and elements of the decision set are control parameters, comprising ablation voltage level, ablation power, pulse time, pulse amplitude, and pulse frequency; and
constructing the decision matrix V according to the effect of the elements in the signal data set Son the output of the decision set D, as shown in Table 1:

Table 1

| S\D | $d_1$ | $d_2$ | $\cdots$ | $d_m$ |
|-----|-------|-------|----------|-------|
| $s_1$ | $v_{11}$ | $v_{12}$ | $\cdots$ | $v_{1m}$ |
| $s_2$ | $v_{21}$ | $v_{22}$ | $\cdots$ | $v_{2m}$ |
| $\vdots$ | $\vdots$ | $\vdots$ | $\ddots$ | $\vdots$ |
| $s_n$ | $v_{n1}$ | $v_{n2}$ | $\cdots$ | $v_{nm}$ |

wherein, $v_{ij}$ represents a decision matrix factor, i represents a number of a sensor, i E [1,2,3 ..., n], j represents a number of a decision output, and $j \in [1,2,3 ..., m]$;

S2-2. normalizing V, summing up each row, that is, $W_i = \dfrac{v_{i1}}{\sum_{i=1}^{n} v_{i1}} + \dfrac{v_{i2}}{\sum_{i=1}^{n} v_{i2}} + \cdots + \dfrac{v_{im}}{\sum_{i=1}^{n} v_{im}}$ , combining $w_i$

into the sensor coefficient matrix W, and normalizing W, that is, $w_i = \dfrac{w_i}{\sum_{i=1}^{n} w_i}$ is the coefficient of an $i^{th}$ sensor;

S2-3. for the comprehensive environment in which the sample data is located, constructing the corresponding relationship matrix R for the single environment respectively according to Cauchy distribution, wherein $r_{ij}$ is an element in a single environment matrix,

$$r_{ij}(s_i) = \frac{1}{1 + \alpha(s_i - a_{ij})^\beta}$$

wherein $\alpha$ and $\beta$ are empirical parameters, $s_i$ is sensor signal data numbered i, and $a_{ij}$ is a threshold value of a decision output numbered j corresponding to a sensor numbered i in the single environment; and

S2-4. fusing the sensor coefficient matrix W obtained in the step S2-2 and the relationship matrices R of the k single environments obtained in the step S2-3 respectively, that is, F=WR, wherein F is the fusion results in the single environment, that is,

$$F = \begin{bmatrix} w_1 & \cdots & w_n \end{bmatrix} \begin{bmatrix} r_{11} & \cdots & r_{1m} \\ \vdots & \ddots & \vdots \\ r_{n1} & \cdots & r_{nm} \end{bmatrix}.$$

Furthermore, $\alpha$ = 1, and $\beta$ = 2.

Furthermore, the threshold value $a_{ij}$ is set according to a resistivity of an environment in which the target substance is located.

A system for artificial intelligence-based radiofrequency ablation parameter optimization and information synthesis, the system comprising a radiofrequency ablation controller, a plasma wand, and a controlling foot switch, wherein a variety of sensors are arranged on a knife head of the plasma wand and are configured to continuously collect data of a target substance and a surrounding environment of the target substance in real time, and transfer the data to the radiofrequency ablation controller;

Both wired and wirelessconnection ofthe controlling foot switch and the radiofrequency ablation controllercould be designed, and the controlling foot switch is used for a user to control an output of a radiofrequency signal; and the radiofrequency ablation controller outputs a control signal to the plasma wand, to adjust a control parameter.

Beneficial effects of the present invention are as follows.

A multi-sensor adaptive plasma radiofrequency ablation operation system of the present invention senses and collects signals of a lesion tissue and a surrounding environment of the lesion tissue through an environmental sensor, and may provide information such as the position of the lesion tissue and the size of an ablation range with reference to an artificial neural network, which is helpful for doctors to determine the condition of the lesion tissue and control energy transfer during the ablation process, thereby helping to reduce secondary trauma.

Other features and advantages of the present invention will be described in detail in the detailed description which follows.

## BRIEF DESCRIPTION OF THE DRAWINGS

Through a more detailed description of exemplary embodiments of the present invention in combination with the drawings, the above and other objectives, features, and advantages of the present invention are more obvious. In the exemplary embodiments of the present invention, the same reference numerals generally represent the same components.

FIG. 1 is a schematic structural diagram of the present invention.

FIG. 2 is a schematic diagram of an algorithm of an artificial neural network according to the present invention.

FIG. 3 shows variation curves of voltage, power, and temperature according to the present invention.

FIG. 4 is a schematic diagram of a comparison between a voltage change in an adaptive ablation process and a voltage change in a conventional ablation process according to an embodiment of the present invention.

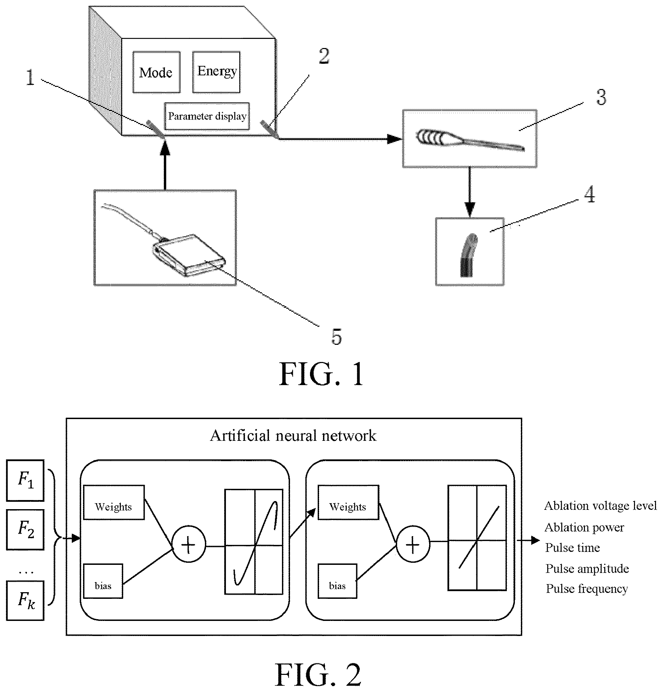

In FIG. 1: 1. Input interface; 2. Output interface; 3. Plasma wand; 4. Knife head; and 5. Foot Switch.

## DETAILED DESCRIPTION OF THE EMBODIMENTS

Exemplary embodiments of the present invention are described in more detail below with reference to the accompanying drawings. Although the accompanying drawings show the exemplary embodiments of the present invention, it should be understood that the present invention may be implemented in various manners and is not limited by the embodiments described herein.

A method for artificial intelligence-based radiofrequency ablation parameter optimization and information synthesis is provided. The method is applied to a radiofrequency ablation controller including a processor and an artificial intelligence module.

S1. The processor of the radiofrequency ablation controller preprocesses sample data, where the sample data is a sensor parameter of a target substance, and the processor sends the preprocessed sample data to the artificial intelligence module.

S2. The artificial intelligence module performs fuzzy computing on the preprocessed sample data and radiofrequency ablation control parameters for the sample data, and establishes an artificial neural network model, where the control parameters include ablation occurrence time, ablation trigger time, and energy frequency. The fuzzy computing further includes:

S2-1. Construct a sensor signal data set $S$ for the sample data; construct a decision set of the controller; and construct a decision matrix V according to an effect of elements in the signal data set Son an output of the decision set D.

S2-2. Normalize V, sum up each row, combine the summed data into a sensor coefficient matrix W, and normalize W.

S2-3. For a comprehensive environment in which the sample data is located, construct a corresponding relationship matrix R for a single environment respectively according to Cauchy distribution, where the quantity of the relationship matrices is k.

S2-4. Fuse the sensor coefficient matrix W and the relationship matrices R of k single environments respectively, that is, F=WR, to obtain fusion results of k single collection environments, $[F_1, F_2, ..., F_k]$.

S2-5. Input the fusion results $[F_1, F_2, ..., F_k]$ of k single tissue environments of a plurality of groups of samples and the radiofrequency ablation control parameters corresponding to each sample into an artificial neural network according to the steps S2-1 to S2-4, as shown in FIG. 2, and set a quantity of layers of the network for self-learning, to obtain a single tissue environment factor corresponding to the sample data and the established artificial neural network model.

During specific implementation, the step S2 is:

S2-1. Construct the sensor signal data set $S=[s_1, s_2, ..., s_n]$, where n is the quantity of sensors;

construct the decision set $D=[d_1, d_2, ..., d_m]$ of the controller based on types andproperty of the sensors, where m is the quantity of decision results, and elements of the decision set are control parameters, including ablation voltage level, ablation power, pulse time, pulse amplitude, and pulse frequency; and

construct the decision matrix V according to the effect of the elements in the signal data set Son the output of the decision set D, as shown in Table 1:

Table 1

| S\D | $d_1$ | $d_2$ | ⋯ | $d_m$ |
|-----|-------|-------|-----|-------|
| $s_1$ | $v_{11}$ | $v_{12}$ | ⋯ | $v_{1m}$ |
| $s_2$ | $v_{21}$ | $v_{22}$ | ⋯ | $v_{2m}$ |
| ⋮ | ⋮ | ⋮ | ⋱ | ⋮ |
| $s_n$ | $v_{n1}$ | $v_{n2}$ | ⋯ | $v_{nm}$ |

where, $v_{ij}$ represents a decision matrix factor, i represents a number of a sensor, i E [1,2,3 ..., n], j represents a number of a decision output, and j $\in$ [1,2,3 ..., m].

S2-2. Normalize V, sum up each row, that is, $W_i = \dfrac{v_{i1}}{\sum_{i=1}^{n} v_{i1}} + \dfrac{v_{i2}}{\sum_{i=1}^{n} v_{i2}} + \cdots + \dfrac{v_{im}}{\sum_{i=1}^{n} v_{im}}$ , combine $w_i$ into the sensor

coefficient matrix W, and normalize W, that is, $W_i = \dfrac{w_i}{\sum_{i=1}^{n} w_i}$ is the coefficient of an $i^{th}$ sensor.

S2-3. For the comprehensive environment in which the sample data is located, construct the corresponding relationship matrix R for the single environment respectively according to Cauchy distribution, where $r_{ij}$ is an element in a single environment matrix,

$$r_{ij}(s_i) = \frac{1}{1 + \alpha(s_i - a_{ij})^\beta}$$

where, $\alpha$ and $\beta$ are empirical parameters, $s_i$ is sensor signal data numbered i, and $a_{ij}$ is a threshold value of a decision output numbered j corresponding to a sensor numbered i in the single environment.

S2-4. Fuse the sensor coefficient matrix W obtained in the step S2-2 and the relationship matrices R of the k single environments obtained in the step S2-3 respectively, that is, F=WR, where F is the fusion results in the single environment, that is,

$$F=[w_1 \quad \cdots \quad w_n]\begin{bmatrix} r_{11} & \cdots & r_{1m} \\ \vdots & \ddots & \vdots \\ r_{n1} & \cdots & r_{nm} \end{bmatrix}.$$

S3. The processor preprocesses signals collected by sensors on a plasma wand and sends the preprocessed signals to the artificial intelligence module.

S4. The artificial intelligence module imports preprocessed sensor data into the artificial neural network model established in the step S2 for analysis and fusion, to obtain results.

Further, the method is applied to the radiofrequency ablation controller. A signal input end of the radiofrequency ablation controller is connected to a signal output end of each sensor on the plasma wand, and the radiofrequency ablation controller outputs a control signal to the plasma wand, to adjust the control parameters, so that auxiliary environmental analysis can be performed in clinical practice and simulation training, and matching operating parameters can be provided to doctors for reference.

Further, the sensors include, but are not limited to voltage, current, impedance, temperature, humidity, contact force, and the like.

Further, in the steps S1 and S3, the preprocessing further includes:

S1-1. Amplify and filter sensor signal data. The filtering includes high-pass, low-pass, and band-pass filtering; and the high-pass and low-pass filtering can effectively reduce noise, and the band-pass filtering can remove a non-physiological spurious signal.

S1-2. Perform signal adjustment including linear and nonlinear correction on the filtered signal data. The common linear correction is to use an offset to correct a signal, such as an adjustment of an ambient temperature plus or minus 3°. For the nonlinear correction, linear and nonlinear correction can be performed on a signal in the form of piecewise fitting. Generally, polynomial and analytic functions $\frac{1}{x}$, $e^x$, lgx, and the like are used. Through sensor input data, environmental parameters, and sensor output data, the least squares method is used to obtain a fitting function to be approximated to replace a nonlinear relationship, thereby compensating for a nonlinear loss of a sensor signal, and obtaining fidelity sensor signal data.

Further, the threshold value $a_{ij}$ is set according to a human body resistance comparison table (Table 1) and a frequency comparison table (Table 2).

Table 1

| Direct-current resistivity of human tissues ($\Omega$ m) | | | | | |
|---|---|---|---|---|---|
| Blood | Nerve | Liver | Muscle | Fat | Membranous bone |
| 1.85 | 25 | 80 | 90 | 1,080 | 2,000,000 |

Table 2

| Relationship between resistivity and frequency of human muscle tissues | | | | | |
|---|---|---|---|---|---|
| Frequency | 0 | 100 Hz | 10 kHz | 10 MHz | 10 GHz |
| Resistivity ($\Omega$ m) | 90 | 9.1 | 7.7 | 2.0 | 0.8 |

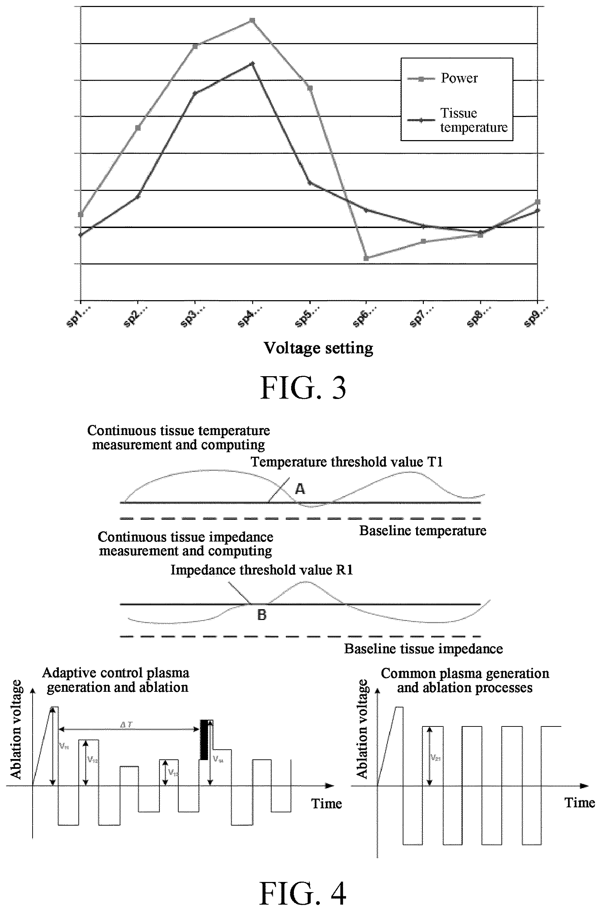

According to the fusion results, output current, voltage, power, and other parameters can be adaptively adjusted at a specific temperature according to characteristics of different tissues of the human body, to achieve needs of optimal surgery and lesion tissue configuration parameters. According to voltage setting values and variation curves of power and temperature, as shown in FIG. 4, in a range of 100 V to 350 V of a plasma generator, an auxiliary prompt is given to recommend using 100 kHz square wave to adapt to power of 100 W to 400 W, and using 200 kHz, 300 kHz, 400 kHz high-frequency harmonics for blood coagulation at the power of 100 W. Cutting of a cartilage may be finely controlled at 100 microns to 120 microns.

A multi-sensor adaptive plasma ablation system is provided. The system includes a radiofrequency ablation controller, a plasma wand, and a controlling foot switch.

A variety of sensors are arranged on a knife head of the plasma wand and are configured to continuously collect data of a lesion tissue and a surrounding environment of the lesion tissue in real time, and transfer the data to the radiofrequency ablation controller.

Both wired and wireless connection of the controlling foot switch and the radiofrequency ablation controller could be designed, and the controlling foot switch is used for a user to control an output of a radiofrequency signal.

The radiofrequency ablation controller outputs a control signal to a display interface of the plasma wand for auxiliary reference of the user in control of the output current and voltage.

The embodiments of the present invention are described above, and the foregoing descriptions are exemplary but not exhaustive and are not limited to the disclosed embodiments. Without departing from the scope and spirit of the described embodiments, many modifications and variations are apparent to a person of ordinary skill in the technical field.

**Claims**

1. A method for artificial intelligence-based radiofrequency ablation parameter optimization and information synthesis, the method being applied to a radiofrequency ablation controller comprising a processor and an artificial intelligence module; and the method comprising

   S1. preprocessing, by the processor of the radiofrequency ablation controller, sample data, wherein the sample data is sensor parameters of a target substance and a surrounding environment of the target substance, and sending, by the processor, the preprocessed sample data to the artificial intelligence module;

   S2. performing, by the artificial intelligence module, fuzzy computing on the preprocessed sample data and radiofrequency ablation control parameters for the sample data, and establishing an artificial neural network model, wherein the control parameters comprise ablation occurrence time, ablation trigger time, and energy frequency;

   S3. preprocessing, by the processor, signals collected by sensors on a plasma wand and sends the preprocessed signals to the artificial intelligence module; and

   S4. importing, by the artificial intelligence module, preprocessed sensor data into the artificial neural network model established in the step S2 for analysis and fusion, to obtain the radiofrequency ablation control parameters.

2. The method for artificial intelligence-based radiofrequency ablation parameter optimization and information synthesis according to claim 1, the method being applied to the radiofrequency ablation controller, wherein a signal input end of the radiofrequency ablation controller is connected to a signal output end of each sensor on the plasma wand, and the radiofrequency ablation controller outputs a control parameter of the plasma wand.

3. The method for artificial intelligence-based radiofrequency ablation parameter optimization and information synthesis according to claim 1, wherein the sensors comprise voltage, current, impedance, temperature, humidity, contact force, and the like.

4. The method for artificial intelligence-based radiofrequency ablation parameter optimization and information synthesis according to claim 1, wherein in the steps S1 and S3, the preprocessing further comprises:

S1-1. amplifying and filtering sensor signal data; and

S1-2. performing signal adjustment comprising linear and nonlinear correction on the filtered signal data.

5. The method for artificial intelligence-based radiofrequency ablation parameter optimization and information synthesis according to claim 1, wherein in the step S2, the fuzzy computing further comprises:

S2-1. constructing a sensor signal data set $S$ for the sample data; constructing a decision set of the controller; and constructing a decision matrix V according to an effect of elements in the signal data set Son an output of the decision set D;

S2-2. normalizing V, summing up each row, combining the summed data into a sensor coefficient matrix W, and normalizing W;

S2-3. for a comprehensive environment in which the sample data is located, constructing a corresponding relationship matrix R for a single environment respectively according to Cauchy distribution, wherein the quantity of the relationship matrices is k;

S2-4. fusing the sensor coefficient matrix W and the relationship matrices R of k single environments respectively, that is, F=WR, to obtain fusion results of k single collection environments, $[F_1, F_2, ..., F_k]$; and

S2-5. inputting the fusion results $[F_1, F_2, ..., F_k]$ of k single tissue environments of a plurality of groups of samples and the radiofrequency ablation control parameters corresponding to each sample into an artificial neural network according to the steps S2-1 to S2-4, and setting a quantity of layers of the network for self-learning, to obtain a single tissue environment factor corresponding to the sample data and the established artificial neural network model.

6. The method for artificial intelligence-based radiofrequency ablation parameter optimization and information synthesis according to claim 5, wherein in the step S2, the fuzzy computing further comprises:

S2-1. constructing the sensor signal data set $S=[s_1, s_2, ..., s_n]$, wherein n is the quantity of sensors; constructing the decision set $D=[d_1, d_2, ..., d_m]$ of the controller based on types and natures of the sensors, wherein m is the quantity of decision results, and elements of the decision set are control parameters, comprising ablation voltage level, ablationpower, pulse time, pulse amplitude, and pulse frequency; and constructing the decision matrix V according to the effect of the elements in the signal data set Son the output of the decision set D, as shown in Table 1:

Table 1

| S\D | $d_1$ | $d_2$ | ⋯ | $d_m$ |
|---|---|---|---|---|
| $s_1$ | $v_{11}$ | $v_{12}$ | ⋯ | $v_{1m}$ |
| $s_2$ | $v_{21}$ | $v_{22}$ | ⋯ | $v_{2m}$ |
| ⋮ | ⋮ | ⋮ | ⋱ | ⋮ |
| $s_n$ | $v_{n1}$ | $v_{n2}$ | ⋯ | $v_{nm}$ |

wherein, $v_{ij}$ represents a decision matrix factor, i represents a number of a sensor, i E [1,2,3 ..., n], j represents a number of a decision output, and $j \in [1,2,3 ..., m]$;

S2-2. normalizing V, summing up each row, that is, $W_i = \dfrac{v_{i1}}{\sum_{i=1}^{n} v_{i1}} + \dfrac{v_{i2}}{\sum_{i=1}^{n} v_{i2}} + \cdots + \dfrac{v_{im}}{\sum_{i=1}^{n} v_{im}}$, combining $w_i$ into the sensor coefficient matrix W, and normalizing W, that is, $w_i = \dfrac{w_i}{\sum_{i=1}^{n} w_i}$ is the coefficient of an $i^{th}$ sensor;

S2-3. for the comprehensive environment in which the sample data is located, constructing the corresponding relationship matrix R for the single environment respectively according to Cauchy distribution, wherein $r_{ij}$ is an element in a single environment matrix,

$$r_{ij}(s_i) = \frac{1}{1 + \alpha(s_i - a_{ij})^\beta}$$

wherein $\alpha$ and $\beta$ are empirical parameters, $s_i$ is sensor signal data numbered i, and $a_{ij}$ is a threshold value of a decision output numbered j corresponding to a sensor numbered i in the single environment; and

S2-4. fusing the sensor coefficient matrix W obtained in the step S2-2 and the relationship matrices R of the k single environments obtained in the step S2-3 respectively, that is, F=WR, wherein F is the fusion results in the single environment, that is,

$$F = \begin{bmatrix} w_1 & \cdots & w_n \end{bmatrix} \begin{bmatrix} r_{11} & \cdots & r_{1m} \\ \vdots & \ddots & \vdots \\ r_{n1} & \cdots & r_{nm} \end{bmatrix}.$$

7. The method for artificial intelligence-based radiofrequency ablation parameter optimization and information synthesis according to claim 6, wherein $\alpha$ = 1, and $\beta$ = 2.

8. The method for artificial intelligence-based radiofrequency ablation parameter optimization and information synthesis according to claim 6, wherein the threshold value $a_{ij}$ is set according to a resistivity of an environment in which the target substance is located.

9. A system for artificial intelligence-based radiofrequency ablation parameter optimization and information synthesis, the system comprising a radiofrequency ablation controller, a plasma wand, and a controlling foot switch, wherein

a variety of sensors are arranged on a knife head of the plasma wand and are configured to continuously collect data of a target substance and a surrounding environment of the target substance in real time, and transfer the data to the radiofrequency ablation controller;

Both wired and wireless connection of the controlling foot switch and the radiofrequency ablation controller could be designed, and the controlling foot switch is used for a user to control an output of a radiofrequency signal; and

the radiofrequency ablation controller outputs a control signal to the plasma wand, to adjust a control parameter.

Mode | Energy

Parameter display

1

2

3

4

5

FIG. 1

Artificial neural network

$F_1$

$F_2$

...

$F_k$

Weights

bias

Weights

bias

Ablation voltage level
Ablation power
Pulse time
Pulse amplitude
Pulse frequency

FIG. 2

Voltage setting

FIG. 3

FIG. 4

Europäisches
Patentamt

European
Patent Office

Office européen
des brevets

# EUROPEAN SEARCH REPORT

Application Number

EP 22 18 4602

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/265309 A1 (WHAM ROBERT H [US] ET AL) 20 August 2020 (2020-08-20) * abstract; claims 1-20; figures 1-10 * * paragraphs [0058] - [0118] * | 1-9 | INV. A61B18/12 ADD. A61B18/00 A61B5/00 |
| X A | WO 2018/217798 A2 (BOSTON SCIENT SCIMED INC [US]) 29 November 2018 (2018-11-29) * abstract; claims 1-15; figures 1-4 * * paragraphs [0050] - [0086] * | 9 1-8 | |
| X | US 2017/007308 A1 (MUN JOUNG HWAN [KR] ET AL) 12 January 2017 (2017-01-12) * abstract; claims 1-15; figures 1-7 * * paragraphs [0038] - [0087] * | 1-9 | |

TECHNICAL FIELDS
SEARCHED      (IPC)

A61B
G16H
G06N

The present search report has been drawn up for all claims

| Place of search | Date of completion of the search | Examiner |
|---|---|---|
| Munich | 14 December 2022 | Mendelevitch, L |

EPO FORM 1503 03.82 (P04C01)

## ANNEX TO THE EUROPEAN SEARCH REPORT
## ON EUROPEAN PATENT APPLICATION NO.

EP 22 18 4602

This annex lists the patent family members relating to the patent documents cited in the above-mentioned European search report.
The members are as contained in the European Patent Office EDP file on
The European Patent Office is in no way liable for these particulars which are merely given for the purpose of information.

14-12-2022

| Patent document cited in search report | | Publication date | Patent family member(s) | | Publication date |
|---|---|---|---|---|---|
| US 2020265309 | A1 | 20-08-2020 | NONE | | |
| WO 2018217798 | A2 | 29-11-2018 | CN | 110891508 A | 17-03-2020 |
| | | | EP | 3629964 A2 | 08-04-2020 |
| | | | US | 2018338703 A1 | 29-11-2018 |
| | | | WO | 2018217798 A2 | 29-11-2018 |
| US 2017007308 | A1 | 12-01-2017 | KR | 20170007590 A | 19-01-2017 |
| | | | US | 2017007308 A1 | 12-01-2017 |

EPO FORM P0459

For more details about this annex : see Official Journal of the European Patent Office, No. 12/82